(19) Europäisches Patentamt
European Patent Office
Office européen des brevets

(11) Publication number: **0 084 860**
**A1**

(12) **EUROPEAN PATENT APPLICATION**

(21) Application number: **83100478.3**

(22) Date of filing: **20.01.83**

(51) Int. Cl.³: **C 07 C 21/22**, C 07 C 17/10 // C07C49/203

(30) Priority: **26.01.82 JP 10677/82**

(43) Date of publication of application: **03.08.83**
**Bulletin 83/31**

(84) Designated Contracting States: **CH DE FR GB LI NL**

(71) Applicant: **Shin-Etsu Chemical Co., Ltd., 6-1, Otemachi 2-chome, Chiyoda-ku, Tokyo (JP)**

(72) Inventor: **Toshinobu, Ishihara, 2-1-3-3, Minato-cho, Joetsu-shi, Niigata-ken (JP)**
Inventor: **Akira, Yamamoto, 2-1-3-13, Minato-cho, Joetsu-shi, Niigata-ken (JP)**

(74) Representative: **Blum, Rudolf Emil Ernst et al, c/o E. BLUM & CO. Vorderberg 11, CH-8044 Zürich (CH)**

(54) **A novel compound 5-chloro-1-bromo-1-pentyne and a method for the preparation thereof.**

(57) A novel organic compound 5-chloro-1-bromo-1-pentyne is disclosed. This compound is prepared by the reaction of 5-chloro-1-pentyne with potassium hypobromite in an aqueous medium at a temperature of 10 to 40 °C in a high yield. The compound is useful as an intermediate for the synthesis of Z-6-heneicosen-11-one which is known as a sex pheromone compound of a noxious insect Dougals-fir tussock moth notorious in the western North-American forests.

EP 0 084 860 A1

0084860

# A NOVEL COMPOUND 5-CHLORO-1-BROMO-1-PENTYNE AND A METHOD FOR THE PREPARATION THEREOF

## BACKGROUND OF THE INVENTION

The present invention relates to a novel compound 5-chloro-1-bromo-1-pentyne and a method for the preparation thereof.

As is well known, a method is under rapid growing for the extermination or control of pests or noxious insects in the fields by use of a sex pheromone of the respective insect and the chemical structures of several sex pheromone compounds have already been established. For example, the sex pheromone excreted by Douglas-fir tussock moth causing great damages to the forests in western North America is chemically Z-6-heneicosen-11-one and it is eagerly desired to develop a convenient and efficient method for the synthesis of this compound. The inventors have also undertaken extensive investigations with such an object and, in the course of their experimental works, they arrived at a discovery of a novel compound which is useful as an intermediate for the synthesis of the above mentioned sex pheromone compound.

## SUMMARY OF THE INVENTION

The novel compound of the present invention, which is useful as an intermediate for the industrial production of

the sex pheromone compound Z-6-heneicosen-11-one by a synthetic method, is 5-chloro-1-bromo-1-pentyne expressed by the structural formula $BrC{\equiv}C\text{-}CH_2\text{-}CH_2\text{-}CH_2Cl$ and not described in any prior art literatures.

The novel compound of the invention 5-chloro-1-bromo-1-pentyne can readily be obtained chemically by the reaction of potassium hypobromite and 5-chloro-1-pentyne in an aqueous reaction medium.

DETAILED DESCRIPTION OF THE PREFERRED EMBODIMENTS

As is mentioned above, the 5-chloro-1-bromo-1-pentyne as the compound of the invention can be synthesized by adding 5-chloro-1-pentyne into an aqueous solution of potassium hypobromite, which can be obtained by introducing bromine into an aqueous solution of potassium hydroxide in a concentration of about 30 %, under agitation to effect the reaction at about 10 to 40 °C followed by the phase separation of the reaction mixture and distillatin of the organic phase under reduced pressure. The yield of the desired compound 5-chloro-1-bromo-1-pentyne in the above described procedure is usually about 85 % of the theoretical value. The reaction is expressed by the following reaction equation:

$$KOBr + HC{\equiv}C\text{-}CH_2\text{-}CH_2\text{-}CH_2Cl \longrightarrow BrC{\equiv}C\text{-}CH_2\text{-}CH_2\text{-}CH_2Cl + KOH.$$

5-Chloro-1-pentyne as the starting material in the above

reaction is a known compound and can be obtained readily, for example, by the reaction of 1-bromo-3-chloropropane with sodium acetylide in liquid ammonia.

As is mentioned above, 5-chloro-1-bromo-1-pentyne of the present invention is a useful compound as an intermediate for the synthetic preparation of Z-6-heneicosen-11-one. That is, 5-chloro-1-bromo-1-pentyne is reacted with n-pentylmagnesium chloride, i.e. the Grignard reagent of n-pentyl chloride, to form 4-decynyl chloride which can be converted to Z-4-decenyl chloride by the partial hydrogenation reaction. The desired Z-6-heneicosen-11-one can be obtained by the reaction of Z-4-decenylmagnesium chloride, i.e. the Grignard reagent of the above obtained Z-4-decenyl chloride, with undecylic acid anhydride.

Z-6-Heneicosen-11-one is the sex pheromone compound excreted by the pests Douglas-fir tussock moth causing great damages in the forests in the western North America and can be usd as an exterminating or controlling agent for the insects since even a trace amount of the compound floating in the air may disturb the intersexual communication of the insect species.

In the following, an example is given for the synthesis and identification of the inventive compound 5-chloro-1-bromo-1-pentyne and the synthetic preparation of

Z-6-heneicosen-11-one starting with the inventive compound as the intermediate.

Example.

Into a 400 ml portion of an aqueous solution containing 120 g of potassium hydroxide dissolved therein kept at 10 to 20 °C were added dropwise 150 g of bromine under agitation to effect the reaction between them. Thereafter, 60 g of 5-chloro-1-pentyne were added to this reaction mixture containing the above obtained potassium hypobromite and the reaction of them was performed in an atmosphere of air with agitation at a reaction temperature of 20 °C for 48 hours. After the end of the above reaction time, the reaction mixture was subjected to the phase separation and the organic solution in the upper layer was distilled under reduced pressure to give 90.3 g of a fraction boiling at 62 °C under a pressure of 2 mmHg as the reaction product.

This product was analyzed by mass spectrometry to give the results shown below, from which this product was identified to be the desired 5-chloro-1-bromo-1-pentyne.

Mass spectrometric data: m/e (relative intensity of the peak)

180*° (13); 145* (7); 117* (19); 101° (15); 65 (100); 51 (13); 39 (31)

The peaks of the m/e value marked with * are for the species having the bromine isotope $^{79}Br$ and each accompanied by the peak for the species having the bromine isotope $^{81}Br$.

The peaks of the m/e value marked with ° are for the species having the chlorine isotope $^{35}Cl$ and each accompanied by the peak for the species having the chlorine isotope $^{37}Cl$.

Following is a description of the preparation of Z-6-heneicosen-11-one from the above obtained 5-chloro-1-bromo-1-pentyne.

A tetrahydrofuran solution of a Grignard reagent n-pentylmagnesium chloride was prepared by the dropwise addition of 53.3 g (0.5 mole) of n-pentyl chloride into a mixture of 12 g of magnesium metal, 1 mg of iodine and 300 ml of tetrahydrofuran at a temperature of 40 to 50 °C under a stream of nitrogen gas.

Into another reaction vessel were taken 91 g (0.5 mole) of 5-chloro-1-bromo-1-pentyne, 300 ml of tetrahydrofuran and 7.5 g of copper(I) bromide to form a reaction mixture, into which the solution of n-pentylmagnesium chloride in tetrahydrofuran prepared above was added dropwise at a temperature of 5 to 10 °C under a stream of nitrogen gas. After completion of the dropwise addition of the tetrahydrofuran

solution, agitation was further continued for additional one hour at 20 °C and then the reaction mixture was subjected to hydrolysis in an aqueous solution of ammonium chloride and hydrogen chloride followed by phase separation of the mixture. The organic phase thus taken was concentrated by distillation to give 51.8 g of 4-decynyl chloride, which amount corresponded to about 60 % yield of the theoretical value.

The above obtained 4-decynyl chloride was converted to Z-4-decenyl chloride by the partial hydrogenation reaction in methyl alcohol in the presence of a palladium catalyst supported on barium sulfate and this Z-4-decenyl chloride was reacted with magnesium metal in tetrahydrofuran to give a Grignard reagent Z-4-decenylmagnesium chloride which was further reacted with undecylic acid anhydride to give Z-6-heneicosen-11-one.

WHAT IS CLAILMED IS:

1. 5-Chloro-1-bromo-1-pentyne.

2. A method for the preparation of 5-chloro-1-bromo-1-pentyne which comprises admixing 5-chloro-1-pentyne with an aqueous solution of potassium hypobromite to effect the reaction between them.

3. The method as claimed in claim 2 wherein the reaction of 5-chloro-1-pentyne with potassium hypobromite in the aqueous solution is carried out at a temperature in the range from about 10 to about 40 °C.

0084860

European Patent Office

# EUROPEAN SEARCH REPORT

Application number

EP 83 10 0478

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A,P | DE-A-3 120 702 (SHIN-ETSU CHEMICAL CO.) *Claims* | 1-3 | C 07 C 21/22<br>C 07 C 17/10 //<br>C 07 C 49/203 |
| | --- | | |
| A | HOUBEN-WEYL: "Methoden der Organischen Chemie", 4th Edition, vol. V/4, Halogenverbindungen, 1960, Georg Thieme Verlag, pages 217-218, Stuttgart (DE); | 2-3 | |
| | --- | | |
| A | US-A-1 967 864 (R.A.JACOBSON) *The whole document* | 2-3 | |
| | ----- | | |
| | | | TECHNICAL FIELDS SEARCHED (Int. Cl. 3) |
| | | | C 07 C 21/00<br>C 07 C 17/00 |

The present search report has been drawn up for all claims

| Place of search | Date of completion of the search | Examiner |
|---|---|---|
| THE HAGUE | 12-04-1983 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82